Europäisches Patentamt
European Patent Office
Office européen des brevets

(19)

(11) Publication number: **0 405 502 A2**

(12) **EUROPEAN PATENT APPLICATION**

(21) Application number: **90112255.6**

(51) Int. Cl.5: **C07D 249/08**, C07B 57/00

(22) Date of filing: **27.06.90**

(30) Priority: **28.06.89 JP 168079/89**
**29.08.89 JP 222298/89**
**21.09.89 JP 247775/89**
**27.12.89 JP 344736/89**

(43) Date of publication of application:
**02.01.91 Bulletin 91/01**

(84) Designated Contracting States:
**AT BE CH DE DK ES FR GB GR IT LI LU NL SE**

(71) Applicant: **SUMITOMO PHARMACEUTICALS COMPANY, LIMITED**
**2-8, Doshomachi 2-chome, Chuo-ku**
**Osaka-shi Osaka 541(JP)**

(72) Inventor: **Ohashi, Naohito**
**6-5, Takamidai**
**Takatsuki, Osaka(JP)**
Inventor: **Miyauchi, Hiroshi**
**9-35, Miyano-cho**
**Takatsuki, Osaka(JP)**
Inventor: **Shimago, Kozo**
**2-4-2, Tomogaoka**
**Sanda, Hyogo(JP)**

(74) Representative: **Vossius & Partnerner**
**Siebertstrasse 4 P.O. Box 86 07 67**
**D-8000 München 86(DE)**

(54) **Optical resolution of sulfone derivatives.**

(57) A method for the optical resolution of sulfone derivatives, which comprises reacting a mixture of the d-and 1-isomers of a sulfone derivative of the formula:

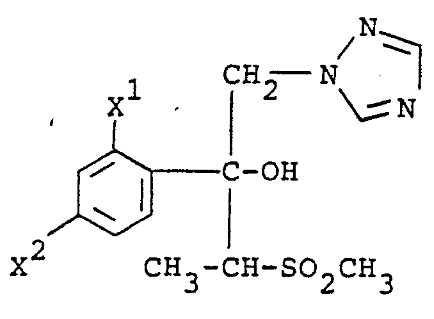

(I)

wherein $X^1$ and $X^2$ are each and independently a hydrogen atom or a halogen atom with optically active 10-camphorsulfonic acid to make a mixture of two kinds of diastereomer salts between them, isolating one of the diastereomer salts from said diastereomer salt mixture on the difference of the diastereomer salts in solubility and decomposing the isolated diastereomer salt.

EP 0 405 502 A2

## OPTICAL RESOLUTION OF SULFONE DERIVATIVES

The present invention relates to the optical resolution of sulfone derivatives. More particularly, it relates to the optical resolution of sulfone derivatives using a certain specific resolving agent.

It is known that sulfone derivatives of the formula:

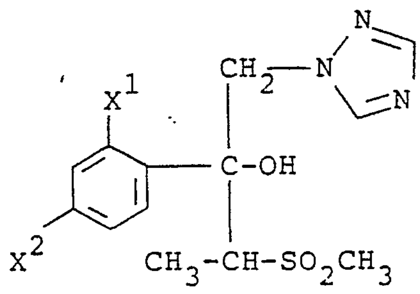

(I)

wherein $X^1$ and $X^2$ are each and independently a hydrogen atom or a halogen atom are useful as antifungal agents (cf. JP-A-57-165370, JP-A-58-185571, JP-A-61-85369), and a strong anti-microbial activity is exhibited by one of their optical isomers (Summary of the 8th Medicinal Chemistry Symposium, 1986, pages 9-12).

In the above significances, the halogen atom represented by $X^1$ and $X^2$ covers fluorine, chlorine, bromine and iodine. Preferred is the case wherein both $X^1$ and $X^2$ are fluorine.

For the synthesis of the optical isomers of the sulfone derivative (I), a diol intermediate is reacted with an optically active acid chloride, the resultant two kinds of diastereomer esters are crystallized, followed by silica gel chromatography for separation, and the thus obtained optically active diol is converted into the corresponding optically active sulfone derivative (Summary of the 8th Medicinal Chemistry Symposium, 1986, pages 9-12). However, this method is not suitable for industrial production, because the separation efficiency of the two kinds of diastereomer esters is not good.

On the other hand, it is known that the sulfone derivative (I) is dissolved into an aqueous, methanolic or aqueous methanolic hydrochloric acid solution of high concentration to make its hydrochloride, while dilution of such solution with water or methanol alone causes the decomposition of the hydrochloride so that the sulfone derivative (I) itself is separated out in a crystalline state. This fact indicates that the sulfone derivative (I) can hardly exist even in the form of hydrochloride, which is usually considered to be quite stable. Therefore, great difficulty was expected to accomplish the resolution of the diastereomer salts as formed between the sulfone derivative (I) and an optically active acid.

As the result of an extensive study, it has now been found that the optical isomers of the sulfone derivative (I) are obtainable with a high efficiency by treatment of a mixture of the d- and 1-isomers of the threo- or erythro-sulfone derivative of the formula (I) with optically active 10-camphorsulfonic acid as a resolvent agent. The present invention is based on the above finding.

According to this invention, a mixture of the d-and I-isomers of the sulfone derivative (I) is reacted with D- or L-10-camphorsulfonic acid in an appropriate solvent to form two kinds of diastereomer salts, these salts are separated into each salt by the utilization of the difference in their solubilities in a suitable solvent, and then the obtained diastereomer salt is decomposed to give the corresponding optically active sulfone derivative (I) having a desired optical configuration.

The sulfone derivative (I) comprises two asymmetric carbon atoms, and therefore a threo-isomer and an erythro-isomer exist. The method of this invention is applicable to both of them, but it is particularly effective for the threo-isomer.

As pointed out by E.L. Eliel in his book "Stereochemistry of Carbon Compounds" (McGraw-Hill Book Company Inc., 1962, New York), "resolution is still very much a matter of trial and error, and even in the papers of experienced investigators one is apt to find, from time to time, a statement that a certain compound resisted resolution by any one of a large number of combinations of resolving agents and solvents that were tried". The success of resolution in the method of this invention entirely owes to the combination of the sulfone derivatve (I) as the sub strate and optically active 10-camphorsulfonic acid as the optical resolving reagent. In fact, the use of a resolving agent having a chemical structure similar to that of D-10-camphorsulfonic acid such as 3-bromo-d-8-camphorsulfonic acid or 3-bromo-d-10-camphorsulfonic acid failed to attain a sufficient resolution efficiency even when the reaction conditions were varied. Also, the

use of a substrate having a chemical structure similar to the sulfone derivative (I) such as (±)-threo-2-(2,4-difluorophenyl)-3-methylthio-1-(1H-1,2,4-triazol-1-yl)butan-2-ol could not attain a high resolution efficiency even under variation of the reaction conditions. Further, the application of various optically active carboxylic acids and phosphoric acids as the resolving agents was examined, but none of them could form salts with the sulfonic derivative (I) under various reaction conditions.

Specifically, the method of the invention may be carried out by dissolving the sulfone derivative (I) and D-10-camphorsulfonic acid or L-10-camphorsulfonic acid in a solvent, followed by preferential crystallization of either one of two kinds of diastereomer salts as formed between them.

As the solvent, there may be used any one chosen from aromatic solvents (e.g. benzene, toluene, chlorobenzene), ethers (e.g. tetrahydrofuran, dioxane), ketones (e.g. acetone, 2-butanone), high polar non-protonic solvents (e.g. acetonitrile, dimethylformamide, dimethylsulfoxide), halogenated hydrocarbons (e.g. dichloromethane, chloroform, dichloroethane), etc. Particularly preferred are the ones chosen from aromatic solvents such as benzene, toluene and chlorobenzene. If desired, their mixtures may be used. For achievement of a high optical purity with a high efficiency, the use of an aromatic solvent as a main solvent is preferred. In any event, the sole use of water or an alcohol is not suitable for the formation of a salt in the method of the invention.

Formation of the salt by dissolving the sulfone derivative (I) in a solvent may be effected at a temperature from room temperature to the boiling point of the solvent, but it is preferred to heat the system to a temperature near the boiling point of the solvent for easy formation of the salt and the preferential crystallization of either one of the diastereomer salts due to the solubility difference. Prior to the separation of the crystallized salt, cooling may be made if necessary, whereby the quantity of the crystallized salt would be increased. Further, a crystalline salt as previously prepared and having a high optical purity may be added to the system as a seed crystal.

D- or L-10-camphorsulfonic acid as the resolving agent may be used in an amount of about 0.5 to 2.0 equivalents, preferably of about 0.7 to 1.0 equivalents, to the substrate.

The optically active camphorsulfonic acid may be used in a hydrated or non-hydrated form. When used in a hydrated form, it is preferably dehydrated, for instance, by azeotropic dehydration prior to the use.

The collected crystals may be, when desired, recrystallized from a solvent as exemplified above, or washed by suspending in such a solvent and filtered so that the optical purity can be increased. The solvent as used for this operation may be the same as or different from the one as used for the salt formation.

In the method of the invention, a mixture of two kinds of diastereomer salts as previously separated may be recrystallized in a solvent as exemplified above or washed by suspending in such solvent and filtering to obtain one of the diastereomer salts.

The diastereomer salt as obtained in the method of the invention, especially (-)-threo-2-(2,4-difluorophenyl)-3-methylsulfonyl-1-(1H-1,2,4-triazol-1-yl)butan-2-ol D-10-camphorsulfonate, exhibits a prominent antifungal activity and is useful as pharmaceutical. The salt may be subjected to salt decomposition by a conventional procedure to give the optically active sulfone derivative (I). For instance, the diastereomer salt is dissolved in an aqueous alkaline solution to transfer optically active 10-camphorsulfonic acid in form of the alkali metal salt into the water layer, and the optically active sulfone derivative is crystallized out, followed by filtration for collection.

After collection of one of the above diastereomer salts, the other diastereomer salt may be recovered from the filtrate as the mother liquor. For instance, the filtrate is concentrated, and the residuse is subjected to salt decomposition in the same manner as above. To the thus obtained sulfone derivative (I), 10-camphorsulfonic acid having an optical activity opposite to that of the previously used optically active 10-camphorsulfonic acid is reacted in the same manner as above to obtain the other optically active isomer of the sulfone derivative (I).

The method of this invention thus enables the production of the d- and l-isomers of the sulfone derivative (I), of which each has a high optical purity in a good yield, from a mixture of those isomers.

Practical and presently preferred embodiments of the invention are illustratively shown in the following Examples wherein % is by weight unless otherwise indicated.

## Example 1

Optical resolution of (±)-threo-2-(2,4-difluorophenyl)-3-methylsulfonyl-1-(1H-1,2,4-triazol-1-yl)butan-2-ol:

Into a reactor, (±)-threo-2-(2,4-difluorophenyl)-3-methylsulfonyl-1-(1H-1,2,4-triazol-1-yl)butan-2-ol (0.50 g), D-10-camphorsulfonic acid monohydrate (0.88 g) and chloroform (10 ml) were charged, and the mixture was heated under reflux to dissolve. The resultant solution was cooled to room temperature, and the chloroform was removed by distillation. The resulting amorphous substance was dissolved in toluene (10 ml) by heating to 90° C and then cooled, whereby crystals separated out at 80° C. After cooling to room temperature, the crystals were colloected by filtration, washed with toluene (20 ml) and dried under reduced pressure to give crystals (0.44 g) mainly comprising (-)-threo-2-(2,4-difluorophenyl)-3-methylsulfonyl-1-(1H-1,2,4-triazol-1-yl)butan-2-ol D-camphor-10-sulfonate.

Optical purity: 82 % e.e. (HPLC analysis).
Melting point: 109 - 115° C (decomp.).
$[\alpha]_D^{25}$ : -13.4° (c = 0.5, $CHCl_3$).

Example 2

Optical resolution of (±)-threo-2-(2,4-difluorophenyl)-3-methylsulfonyl-1-(1H-1,2,4-triazol-1-yl)butan-2-ol:

Into a reactor, (±)-threo-2-(2,4-difluorophenyl)-3-methylsulfonyl-1-(1H-1,2,4-triazol-1-yl)butan-2-ol (11.60 g), D-10-camphorsulfonic acid monohydrate (8.76 g) and toluene (700 ml) were charged, and the mixture was heated under reflux to dissolve. The resultant solution was heated to remove 35 ml of toluene by distillation and cooled to 50° C. After addition of seed crystals of high purity, the resulting mixture was kept hot for 30 minutes, thereby crystals separated. After cooling to room temperature, the crystals were collected by filtration, washed with toluene (100 ml) and dried under reduced pressure to give 10.12 g of (-)-threo-2-(2,4-difluorophenyl)-3-methylsulfonyl-1-(1H-1,2,4-triazol-1-yl)butan-2-ol D-10-camphorsulfonate (10.12 g).

Optical purity: 84 % e.e. (HPLC analysis)
Melting point: 105 - 111° C (decomp.)
$[\alpha]_D^{25}$ : -12.8° (c = 0.50, $CHCl_3$).

The mother liquor as the filtrate was concentrated to give an amorphous substance mainly comprising (+)-threo-2-(2,4-difluorophenyl)-3-methylsulfonyl-1-(1H-1,2,4-triazol-1-yl)butan-2-ol D-10- camphorsulfonate.

Optical purity: 72 % e.e. (HPLC analysis).
Melting point: 50 - 60° C (decomp.).
$[\alpha]_D^{25}$ : +39.6° (c = 1.00, $CHCl_3$).

The crystals of (-)-threo-isomer (9.97 g) as above obtained were dissolved in toluene (200 ml) by heating at 90° C for 3 hours, and the resultant solution was cooled to room temperature. The seprated crystals were collected by filtration, washed with toluene and dried under reduced pressure to give (-)-threo-2-(2,4-difluorophenyl)-3-methylsulfonyl-1-(1H-1,2,4-triazol-1-yl)butan-2-ol D-10- camphorsulfonate.The nuclear magnetic resonance spectrum revealed that the molar ratio of the base and acid in this product was 1 : 1.

Optical purity: 99 % e.e. (HPLC analysis).
Melting point: 109 - 113° C (decomp.).
$[\alpha]_D^{25}$ : -18.8° (c = 0.5, $CHCl_3$).

It was further confirmed by a nuclear magnetic resonance spectrum that the product contained toluene in an amount of 6 %. Elementary analysis showed that the established data corresponds to the theoretical value supporting a molar proportion of (-)-threo-2-(2,4-difluorophenyl)-3-methylsulfonyl-1-(1H-1,2,4-triazol-1-yl)butan-2-ol, D-10-camphorsulfonic acid and toluene being 1 : 1 : 0.5.

The filtrate on purification was concentrated to give an amorphous substance mainly comprising (+)-threo-2-(2,4-difluorophenyl)-3-methylsulfonyl-1-(1H-1,2,4-triazol-1-yl)butan-2-ol D-10-camphor-sulfonate.

Optical purity: 72 % e.e. (HPLC analysis).
Melting point: 52 - 63° C (decomp.).
$[\alpha]_D^{25}$ : +39.2° (c = 1.00, $CHCl_3$).

The above obtained pure (-)-threo-2-(2,4-difluorophenyl)-3-methylsulfonyl-1-(1H-1,2,4-triazol-1-yl)butan-2-ol D-10-camphorsulfonate (2.50 g) was gradually added to a saturated aqueous sodium bicarbonate solution (6 ml) under stirring, and stirring was continued at 40° C for 2 hours. The resultant mixture was ice-cooled, and the precipitated crystals were collected by filtration, washed with ice-water and dried under reduced pressure to give (-)-threo-2-(2,4-difluorophenyl)-3-methylsulfonyl-1-(1H-1,2,4-triazol-1-yl) butan-2-ol (1.34 g). Overall yield, 42.3 %.

Optical purity: 99 % e.e. (HPLC analysis).
Melting point: 147.0 - 148.0 °C.
$[\alpha]_D^{25}$ : -39.3° (c = 1.00, methanol).

| Elementary analysis for $C_{13}H_{15}O_3F_2N_3S$ (%): | |
|---|---|
| Calcd.: | C, 47.13; H, 4.56; N, 12.68; S, 9.68. |
| Found: | C, 47.17; H, 4.49; N, 12.73; S, 9.8. |

Example 3

Optical resolution of (±)-threo-2-(2,4-difluorophenyl)-3-methylsulfonyl-1-(1H-1,2,4-triazol-1-yl)butan-2-ol:

Into a reactor, D-10-camphorsulfonic acid monohydrate (54.4 g) and monochlorobenzene (930 g) were charged, and the resultant mixture was heated to dissolve. After removal of monochlorobenzene (45 g) by distillation, the resulting solution was cooled to 100 °C, (±)-threo-2-(2,4-diflurophenyl)-3-methylsulfonyl-1-(1H-1,2,4-triazol-1-yl)butan-2-ol (80.0 g) was added thereto, and the mixture was heated to dissolve, followed by cooling to 70 °C. Seed crystals of high purity were added thereto at the same temperature, and the resultant mixture was warmed for 30 minutes and then cooled to room temperature. Precipitated crystals were collected by filtration, washed with monochlorobenzene (70 g) two times and dried under reduced pressure to give (-)-threo-2-(2,4-difluorophenyl)-3-methylsulfonyl-1-(1H-1,2,4-triazol-1-yl)butan-2-ol D-10-camphor sulfonate.
Optical purity: 84 % e.e. (HPLC analysis).
Melting point: 114 - 117 °C (decomp.).
$[\alpha]_D^{25}$ : -13.9° (c = 0.5, $CHCl_3$).

The thus obtained product was combined with monochlorobenzene (1470 g) and heated at 100 °C to dissolve, followed by cooling to 75 °C. Seed crystals of high purity were added thereto, and the resultant mixture was kept at the same temperature as above for 30 minutes, followed by cooling to room temperature. Precipitated crystals were collected by filtration, washed with monochlorobenzene (55 g) and dried under reduced pressure to give (-)-threo-2-(2,4-difluorophenyl)-3-methylsulfonyl-1-(1H-1,2,4-triazol1-yl)butan-2-ol D-10-camphorsulfonate.
Optical purity: 97 % e.e. (HPLC analysis).
Melting point: 114 - 118 °C (decomp.).
$[\alpha]_D^{25}$ : -18.8° (c = 0.5, $CHCl_3$).

The above obtained crystals were dissolved in monochlorobenzene (1290 g), and the resultant solution was subjected to the same treatment as above to give (-)-threo-2-(2,4-difluorophenyl)-3-methylsulfonyl-1-(1H-1,2,4-triazol-1-yl)butan-2-ol D-10-camphorsulfonate. The nuclear magnetic resonance spectrum revealed that the molar ratio of the base and acid in this product was 1 : 1.
Optical purity: > 99 % e.e. (HPLC analysis).
Melting point: 116 - 118 °C (decomp.).
$[\alpha]_D^{25}$ : -19.6° (c = 0.5, $CHCl_3$).

It was further confirmed by gas chromatographic analysis that the product contained monochlorobenzene in an amount of 9.2 %. Elementary analysis showed that the established data corresponds to the theoretical value supporting a molar proportion of (-)-threo-2-(2,4-difluorophenyl)-3-methylsulfonyl-1-(1H-1,2,4-triazol-1-yl)butan-2-ol, D-10-camphorsulfonic acid and toluene being 1 : 1 : 0.5.

Furthermore, crystals (59.7 g) as obtained were suspended in water (170 g), and a 9.6 % aqueous sodium hydroxide solution was added thereto to adjust the pH at 7.7. The resultant mixture was stirred at room temperature for 1 hour and ice-cooled. The precipitated crystals were collected by filtration, washed with water (45 g) two times and dried under reduced pressure to give (-)-threo-2-(2,4-difluorophenyl)-3-methylsulfonyl-1-(1H-1,2,4-triazol-1-yl)butan-2-ol (31.3 g).
Optical purity: > 99 % e.e. (HPLC analysis).
Melting point: 147 - 148 °C (decomp.).
$[\alpha]_D^{25}$ : -38.1° (c = 0.5, methanol).

15.6 g of the thus obtained crystals were recrystallized from water to give 14.8 g of (-)-threo-2-(2,4-difluorophenyl)-3-methylsulfonyl-1-(1H-1,2,4-triazol-1-yl)butan-2-ol. Overall yield, 37.2 %.
Optical purity: > 99 % e.e. (HPLC analysis).
Melting point: 147 - 148 °C (decomp.).
$[\alpha]_D^{25}$ : -38.8° (c = 0.5, methanol).
Water content: 0.6 % (measured according to Karl-Fischer method).

| Elementary analysis for $C_{13}H_{15}O_3F_2N_3S.1/9H_2O$ (%): | |
|---|---|
| Calcd.: | C, 46.84; H, 4.60; N, 12.61. |
| Found: | C, 46.77; H, 4.45; N, 12.77. |

Example 4

Optical resolution of (±)-threo-2-(2,4-difluorophenyl)-3-methylsulfonyl-1-(1H-1,2,4-triazol-1-yl)butan-2-ol:

Into a reactor, (±)-threo-2-(2,4-difluorophenyl)-3-methylsulfonyl-1-(1H-1,2,4-triazol-1-yl)butan-2-ol (0.50 g), D-10-camphorsulfonic acid monohydrate (0.38 g) and benzene (30 ml) were charged, and the resultant mixture was heated under reflux to dissolve. The resulting mixture was cooled to 50 °C, seed crystals of high purity were added thereto, and the temperature was kept at 50 °C for 30 minutes, followed by cooling to room temperature. The precipitated crystals were collected by filtration, washed with benzene (20 ml) and dried under reduced pressure to give (-)-threo-2-(2,4-difluorophenyl)-3-methylsulfonyl-1-(1H-1,2,4-triazol-1-yl)butan-2-ol D-10- camphorsulfonate (0.34 g).
Optical purity: 93 % e.e. (HPLC analysis).
Melting point: 108 - 113 °C (decomp.).
$[\alpha]_D^{25}$ : -16.7° (c = 0.50, $CHCl_3$).

Example 5

Optical resolution of (±)-threo-2-(2,4-difluorophenyl)-3-methylsulfonyl-1-(1H-1,2,4-triazol-1-yl)butan-2-ol:

Into a reactor, (±)-threo-2-(2,4-difluorophenyl)-3-methylsulfonyl-1-(1H-1,2,4-triazol-1-yl)butan-2-ol (0.50 g), L-10-camphorsulfonic acid monohydrate (0.38 g) and toluene (10 ml) were charged, and the resultant mixture was heated under reflux to dissolve. The resulting mixture was cooled to 50 °C, seed crystals of high purity were added thereto, and the temperature was kept at 50 °C for 1 hour, followed by cooling to room temperature. The precipitated crystals were collected by filtration, washed with toluene (10 ml) and dried under reduced pressure to give (+)-threo-2-(2,4-difluorophenyl)-3-methylsulfonyl-1-(1H-1,2,4-triazol-1-yl)butan-2-ol L-10-camphorsulfonate.
Optical purity: 65 % e.e. (HPLC analysis).
Melting point: 101 - 108 °C (decomp.).
$[\alpha]_D^{25}$ : +7.27° (c = 0.48, $CHCl_3$).

Example 6

Production of (+)-threo-2-(2,4-difluorophenyl)-3-methylsulfonyl-1-(1H-1,2,4-triazol-1-yl)butan-2-ol:

The amorphous substance (12.79 g) mainly comprising (+)-threo-2-(2,4-difluorophenyl)-3-methylsulfonyl-1-(1H-1,2,4-triazol-1-yl)butan-2-ol D-10-camphorsulfonate was gradually added to a saturated sodium bicarbonate solution (25 ml) warmed at 40 °C under stirring, and stirring was continued at the same temperature for 1.5 hours. After ice-cooling, the precipitated crystals were collected by filtration, washed

with ice-water (20 ml) and dried under reduced pressure to give (+)-threo-2-(2,4-difluorophenyl)-3-methylsulfonyl-1-(1H-1,2,4-triazol-1-yl)butan-2-ol (6.15 g).

Optical purity: 75 % e.e. (HPLC analysis).

Melting point: 148 - 151 °C (decomp.).

$[\alpha]_D^{25}$ : +31.2° (c = 0.2, methanol).

Crystals (5.40 g) thus obtained were combined with L-10-camphorsulfonic acid monohydrate (4.08 g) and toluene (380 ml) and heated under reflux to dissolve. The resulting solution was heated to remove toluene (30 ml) by distillation and then cooled to 80 °C, whereby crystals separated. The temperature was retained at 80 to 85 °C for 3 hours and then cooled to room temperature. The precipitated crystals were collected by filtration, washed with toluene (100 ml) and dried under reduced pressure to give (+)-threo-2-(2,4-difluorophenyl)-3-methylsulfonyl-1-(1H-1,2,4-tirazol-1-yl)butan-2-ol L-10-camphorsulfonate.

Optical purity: 98 % e.e. (HPLC analysis).

Melting point: 109 - 116 °C (decomp.).

$[\alpha]_D^{25}$ : +19.9° (c = 0.50, $CHCl_3$).

The mother liquor as the filtrate was concentrated to give an amorphous substance mainly comprising (-)-threo- 2-(2,4-difluorophenyl)-3-methylsulfonyl-1-(1H-1,2,4-triazol-1-yl)butan-2-ol L-10-camphorsulfonate.

Optical purity: 63 % e.e. (HPLC analysis).

Melting point: 55 - 63 °C (decomp.).

$[\alpha]_D^{25}$ : -35.3° (c = 0.51, $CHCl_3$).

The above obtained crystals (8.12 g) of (+)-threo-isomer were dissolved in toluene (200 ml), and the resultant solution was kept at 90 °C for 3 hours, followed by cooling to room temperature. The precipitated crystals were collected by filtration, washed with toluene (50 ml) and dried under reduced pressure to give (+)-threo-2-(2,4-difluorophenyl)-3-methylsulfonyl-1-(1H-1,2,4-triazol-1-yl)butan-2-ol L-10-camphorsulfonate.

Optical purity: >99 % e.e. (HPLC analysis).

Melting point: 110 - 115 °C (decomp.).

$[\alpha]_D^{25}$ : +20.0° (c = 0.50, $CHCl_3$).

It was confirmed by a nuclear magnetic resonance spectrum that the product contained toluene in an amount of 6 %. Elementary analysis showed that the established data corresponds to the theoretical value supporting a molar proportion of (+)-threo-2-(2,4-difluorophenyl)-3-methylsulfonyl-1-(1H-1,2,4-triazol-1-yl)-butan-2-ol, L-10-camphorsulfonic acid and toluene being 1 : 1 : 0.5.

The above obtained crystals (3.00 g) of (+)-threo-2-(2,4-difluorophenyl)-3-methylsulfonyl-1-(1H-1,2,4-triazol-1-yl)butan-2-ol L-camphor-10-sulfonate were gradually added to a saturated aqueous sodium bicarbonate solution (6 ml) under stirring, and stirring was continued at 40 °C for 2 hours, followed by ice-cooling. The precipitated crystals were collected by filtration, washed with ice-water (3 ml) and dried under reduced pressure to give (+)-threo-2-(2,4-difluorophenyl)-3-methylsulfonyl-1-(1H-1,2,4-triazol-1-yl)butan-2-ol (1.61 g).

Optical purity: >99 % e.e. (HPLC analysis).

Melting point: 146 - 147.0 °C.

$[\alpha]_D^{25}$ : +36.8° (c = 1.00, methanol).

| Elementary analysis for $C_{13}H_{15}O_3F_2N_3S$ (%): | |
|---|---|
| Calcd.: | C, 47.13; H, 4.56; N, 12.68. |
| Found: | C, 47.06; H, 4.61; N, 12.62. |

Reference Example 1

Optical resolution of (±)-threo-2-(2,4-difluorophenyl)-3-methylsulfonyl-1-(1H-1,2,4-triazol-1-yl)butan-2-ol:

(±)-threo-2-(2,4-Difluorophenyl)-3-methylsulfonyl-1-(1H-1,2,4-triazol-1-yl)butan-2-ol (1.33 g), 3-bromo-d-camphor-8-sulfonic acid monohydrate (1.32 g) and monochlorobenzene (100 g) were charged in a reactor, and the resultant mixture was heated for dissolution of the crystals under reflux. The reaction mixture was cooled to 80 °C. Seed crystals of high purity were inoculated thereto at the same temperature, and the

resultant mixture was kept for 1 hour for precipitation of crystals and then cooled to 35 °C. The precipitated crystals were collected by filtration, washed with monochlorobenzene (50 g) and dried under reduced pressure to give 1.54 g of (-)-threo-2-(2,4-difluorophenyl)-3-methylsulfonyl-1-(1H-1,2,4-triazol-1-yl)butan-2-ol 3-bromo-d-camphor-8-sulfonate.
Optical purity: 43 % e.e. (on HPLC analysis).
Melting point: 152 - 167 °C (decomp.).
$[\alpha]_D^{25}$ : +23.8° (c = 0.50, $CHCl_3$).

The filtrate was concentrated to give (+)-threo-2-(2,4-difluorophenyl)-3-methylsulfonyl-1-(1H-1,2,4-triazol1-yl)butan-2-ol 3-bromo-d-camphor-8-sulfonate.
Optical purity: 75 % e.e. (on HPLC analysis).
Melting point: 58 - 68 °C (decomp.).
$[\alpha]_D^{25}$ : +66.3° (c = 0.51, $CHCl_3$).

The thus obtained crystals of (-)-isomer was suspended in monochlorobenzene (75 ml), heated at 90 °C and cooled to 50 °C, followed by collection of the crystals by filtration. Precipitated crystals were washed with monochlorobenzene (30 ml) and dried under reduced pressure to give 1.18 g of (-)-threo-2-(2,4-difluorophenyl)-3-methylsulfonyl-1-(1H-1,2,4-triazol-1-yl)butan-2-ol 3-bromo-d-camphor-8-sulfonate.
Optical purity: 73 % e.e. (on HPLC analysis).
Melting point: 162 - 175 °C (decomp.).
$[\alpha]_D^{25}$ : +12.9° (c = 0.50, $CHCl_3$).

The thus obtained product was further suspended in monochlorobenzene (107 ml) and treated in the same manner as above to give 0.65 g of (-)-threo-2-(2,4-difluorophenyl)-3-methylsulfonyl-1-(1H-1,2,4-triazol-1-yl)butan-2-ol 3-bromo-d-camphor-8-sulfonate.The nuclear magnetic resonance spectrum revealed that the molar ratio of the base and the acid in this product was 1 : 1.
Optical purity: 99 % e.e. (on HPLC analysis).
Melting point: 191 - 194 °C (decomp.).
$[\alpha]_D^{25}$ : -0.2° (c = 0.50, $CHCl_3$).

| Elementary analysis for $C_{23}H_{30}O_7BrF_2N_3S_2$ (%): | |
|---|---|
| Calcd.: | C, 42.99; H, 4.71; N, 6.54; S, 9.98; Br, 12.4. |
| Found: | C, 42.88; H, 4.74; N, 6.49; S, 9.8; Br, 11.9. |

IR ($\nu$ max) $cm^{-4}$: 1745, 1610, 1505, 1295, 1215, 1130, 1030.

The above obtained (-)-threo-2-(2,4-difluorophenyl)-3-methylsulfonyl-1-(1H-1,2,4-triazol-1-yl)butan-2-ol 3-bromo-camphor-8-sulfonate was gradually added to a saturated aqueous sodium bicarbonate solution (1.4 ml) under stirring, and stirring was continued at 40 °C for 1 hour. The reaction mixture was ice-cooled and the precipitated crystals were collected by filtration. The crystals were washed with ice-water (1 ml) and dried under reduced pressure to give 0.31 g of (-)-threo-2-(2,4-difluorophenyl)-3-methylsulfonyl-1-(1H-1,2,4-triazol-1-yl)butan- 2-ol. Overall yield, 23.3 %.
Optical purity: >99 % e.e. (on HPLC analysis).
Melting point: 148.0 - 148.5 °C.
$[\alpha]_D^{25}$ : -38.8° (c = 1.00, MeOH).

| Elementary analysis for $C_{13}H_{15}O_3N_3S$ (%): | |
|---|---|
| Calcd.: | C, 47.18; H, 4.56; N, 12.68; S, 9.68. |
| Found: | C, 46.83; H, 4.57; N, 12.61; S, 9.4. |

Reference Example 2

Optical resolution of (±)-threo-2-(2,4-difluorophenyl)-3-methylsulfonyl-1-(1H-1,2,4-triazol-1-yl)butan-2-ol:

(±)-threo-2-(2,4-Difluorophenyl)-3-methylsulfonyl-1-(1H-1,2,4-triazol-1-yl)butan-2-ol (1.33 g), 3-bromo-d-camphor-10-sulfonic acid monohydrate (140 ml) and benzene (140 ml) were charged in a reactor, and the resultant mixture was heated for dissolution of crystals under reflux. The reaction mixture was cooled to 60 °C. Seed crystals of high purity were inoculated thereto at the same temperature, and the resultant mixture was kept for 1 hour for precipitation of crystals and then cooled to room temperature. Precipitated crystals were collected by filtration, washed with benzene (50 ml) and dried under reduced pressure to give 1.29 g of (-)-threo-2-(2,4-difluorophenyl)-3-methylsulfonyl-1-(1H-1,2,4-triazol-1-yl)butan-2-ol 3-bromo-d-camphor-10-sulfonate.

Optical purity: 69 % e.e. (on HPLC analysis).
Melting point: 109 - 119 °C (decomp.).
$[\alpha]_D^{25}$ : +38.1 ° (c = 0.49, $CHCl_3$).

The filtrate was concentrated to give (+)-threo-2-(2,4-difluorophenyl)-3-methylsulfonyl-1-(1H-1,2,4-triazol-1-yl)butan-2-ol 3-bromo-d-camphor-10-sulfonate.

Optical purity: 67 % e.e. (on HPLC analysis).
Melting point: 57 - 70 °C (decomp.).
$[\alpha]_D^{25}$ : +71.3 ° (c = 0.52, $CHCl_3$).

The thus obtained crystals of (-)-isomer was suspended in benzene (48 ml), heated at 70 °C for 3 hours and cooled to room temperature, followed by collection of crystals by filtration. Precipitated crystals were washed with benzene (30 ml) and dried under reduced pressure to give 1.18 g of (-)-threo-2-(2,4-difluorophenyl)-3-methylsulfonyl-1-(1H-1,2,4-triazol-1-yl)butan-2-ol 3-bromo-d-camphor-10-sulfonate.

Optical purity: 93 % e.e. (on HPLC analysis).
Melting point: 117 - 125 °C (decomp.).
$[\alpha]_D^{25}$ : +28.6 ° (c = 0.50, $CHCl_3$).

The thus obtained product was furter suspended in benzene (63 ml) and treated in the same manner as above to give 0.65 g of (-)-threo-2-(2,4-difluorophenyl)-3-methylsulfonyl-1-(1H-1,2,4-triazol-1-yl)butan-2-ol 3-bromo-d-camphor-10-sulfonate.The nuclear magnetic resonance spectrum revealed that the molar ratio of the base and the acid in this product was 1 : 1.

Optical purity: 95 % e.e. (on HPLC analysis).
Melting point: 121 - 125 °C (decomp.).
$[\alpha]_D^{25}$ : +26.3 ° (c = 0.50, $CHCl_3$).
IR ($\nu$ max) $cm^{-4}$: 1750, 1615, 1500, 1295, 1120, 1040.

It was further confirmed by a nuclear magnetic resonance spectrum that the product contained benzene in a weight proportion of 3.6 %, and an elementary analysis as found was identical to the one obtained by calculation, which showed the molar ratio of (-)-threo-2-(2,4-difluorophenyl)-3-methylsulfonyl-1-(1H-1,2,4-triazol-1-yl)butan-2-ol, 3-bromo-d-camphor-10-sulfonic acid and benzene being 1 : 1 : 0.5.

The above obtained (-)-threo-2-(2,4-difluorophenyl)-3-methylsulfonyl-1-(1H-1,2,4-triazol-1-yl)butan-2-ol 3-bromo-d-camphor-10-sulfonate was gradually added to a saturated aqueous sodium bicarbonate solution (1.4 g) under stirring, and stirring was continued at 40 °C for 1 hour. The reaction mixture was ice-cooled and the precipitated crystals were collected by filtration. The crystals were washed with ice-water (1 ml) and dried under reduced pressure to give 0.30 g of (-)-threo-2-(2,4-difluorophenyl)-3-methylsulfonyl-1-(1H-1,2,4-triazol-1-yl)butan-2-ol. Overall yield, 22.2 %.

Optical purity: 95 % e.e. (on HPLC analysis).
Melting point: 148.0 - 148.5 °C.
$[\alpha]_D^{25}$ : -37.8 ° (c = 1.00, MeOH).

| Elementary analysis for $C_{13}H_{15}F_2N_3S$ (%): | |
|---|---|
| Calcd.: | C, 47.18; H, 4.56; N, 12.68; S, 9.68. |
| Found: | C, 47.08; H, 4.53; N, 12.63; S, 9.4. |

Reference Example 3

Optical resolution of (±)-threo-2-(2,4-difluorophenyl)-3-methylthio-1-(1H-1,2,4-triazol-1-yl)butan-2-ol:

(±)-threo-2-(2,4-Difluorophenyl)-3-methylthio-1-(1H-1,2,4-triazol-1-yl)butan-2-ol (5.99 g), D-camphor-10-sulfonic acid monohydrate (5.ol g) and toluene (36 ml) were charged in a reactor, and the resultant mixture was heated for dissolution of crystals under reflux, during which toluene (6 ml) was removed. The reaction mixture was cooled to 40° C. Seed crystals of high purity were inoculated thereto at the same temperature, and the resultant mixture was kept for 1 hour for precipitation of crystals and then cooled to room temperature. Precipitated crystals were collected by filtration, washed with toluene (10 ml) and dried under reduced pressure to give 4.46 g of (-)-threo-2-(2,4-difluorophenyl)-3-methylthio-1-(1H-1,2,4-triazol-1-yl)butan-2-ol D-camphor-10-sulfonate.

Optical purity: 51 % e.e. (on HPLC analysis).
Melting point: 81 - 90° C (decomp.).
$[\alpha]_D^{25}$ : -18.9° (c = 0.50, $CHCl_3$).

The filtrate was concentrated to give an amorphous substance mainly comprising (+)-threo-2-(2,4-difluorophenyl)-3-methylthio-1-(1H-1,2,4-triazol-1-yl)butan-2-ol D-camphor-10-sulfonate.

Optical purity: 37 % e.e. (on HPLC analysis).
$[\alpha]_D^{25}$ : +41.4° (c = 0.51, $CHCl_3$).

The thus obtained crystals (4.23 g) of (-)-isomer was combined with toluene (30 ml), heated at 50° C for 2 hours and cooled to room temperature, followed by collection of crystals by filtration. Precipitated crystals were washed with toluene (10 ml) and dried under reduced pressure to give 2.86 g of (-)-threo-2-(2,4-difluorophenyl)-3-methylthio-1-(1H-1,2,4-triazol-1-yl)butan-2-ol D-camphor-10-sulfonate. The nuclear magnetic resonance spectrum revealed that the molar ratio of the base and the acid in this product was 1 : 1.

Optical purity: 90 % e.e. (on HPLC analysis).
Melting point: 90 - 96° C (decomp.).
$[\alpha]_D^{25}$ : -38.4° (c = 0.50, $CHCl_3$).

It was further confirmed by a nuclear magnetic resonance spectrum that the product contained toluene in a weight proportion of 6 %, and an elementary analysis as found was identical to the one obtained by calculation, which showed the molar ratio of (-)-threo-2-(2,4-difluorophenyl)-3-methylthio-1-(1H-1,2,4-triazol-1-yl)butan-2-ol, D-camphor-10-sulfonic acid and toluene being 1 : 1 : 1/3.

The above obtained (-)-threo-2-(2,4-difluoro phenyl)-3-methylthio-1-(1H-1,2,4-triazol-1-yl)butan-2-ol D-camphor-10-sulfonate (2.00 g) was gradually added to a saturated aqueous sodium bicarbonate solution (6.0 ml) under stirring, and stirring was continued at 40° C for 1 hour. The reaction mixture was cooled to room temperature and the precipitated crystals were collected by filtration. The crystals were washed with water (10 ml) and dried under reduced pressure to give 1.03 g of (-)-threo-2-(2,4-difluorophenyl)-3-methylthio-1-(1H-1,2,4-triazol-1-yl)butan-2-01, which was recrystallized from a mixture of toluene and heptane to give pure (-)-threo-2-(2,4-difluorophenyl)-3-methylthio-1-(1H-1,2,4-triazol-1-yl)butan-2-ol. Overall yield, 17.6 %.

Optical purity: >99 % e.e. (on HPLC analysis).
Melting point: 134.5 - 135.0° C.
$[\alpha]_D^{25}$ -126.7° (c = 0.51, $CHCl_3$).

| Elementary analysis for $C_{13}H_{15}OF_2N_3S$ (%): | |
|---|---|
| Calcd.: | C, 52.16; H, 5.05; N, 14.04. |
| Found: | C, 52.15; H, 4.98; N, 14.17. |

Reference Example 4

Optical resolution of (±)-threo-2-(2,4-difluorophenyl)-3-methylthio-1-(1H-1,2,4-triazol-1-yl)butan-2-ol:

(±)-threo-2-(2,4-Difluorophenyl)-3-methylthio-1-(1H-1,2,4-triazol-1-yl)butan-2-ol (5.00 g), D-camphor-10-sulfonic acid monohydrate (4.18 g) and xylene (mixture; volume point, 137.5 - 141.5° C) (30 ml) were charged in a reactor, and the resultant mixture was heated for dissolution of crystals under reflux, during which xylene (5 ml) was removed. The reaction mixture was cooled to 35° C. Seed crystals of high purity were inoculated thereto at the same temperature, and the resultant mixture was stirred at room temperature

for 3 hours for precipitation of crystals. Precipitated crystals were collected by filtration, washed with xylene (10 ml) and dried under reduced pressure to give 3.ol g of (-)-threo-2-(2,4-difluorophenyl)-3-methylthio-1-(1H-1,2,4-triazol-1-yl)butan-2-ol D-camphor-10-sulfonate.
Optical purity: 66 % e.e. (on HPLC analysis).
Melting point: 78 - 82° C (decomp.).
$[\alpha]_D^{25}$ : -22.2° (c = 0.50, $CHCl_3$).

The thus obtained crystals (2.86 g) was combined with xylene (15 ml), heated at 50° C for 2 hours and cooled to room temperature, followed by collection of crystals by filtration. Precipitated crystals were washed with xylene (10 ml) and dried under reduced pressure to give 2.04 g of (-)-threo-2-(2,4-difluorophenyl)-3-methylthio-1-(1-1,2,4-triazol-1-yl)butan-2-ol D-camphor-10-sulfonate. The nuclear magnetic resonance spectrum revealed that the molar ratio of the base and the acid in this product was 1 : 1.
Optical purity: 92 % e.e. (on HPLC analysis).
Melting point: 84 - 88° C (decomp.).
$[\alpha]_D^{25}$ : -39.6° (c = 0.50, $CHCl_3$).

It was further confirmed by a nuclear magnetic resonance spectrum that the product contained xylene in a weight proportion of 8 %, and an elementary analysis as found was identical to the one obtained by calculation, which showed the molar ratio of (-)-threo-2-(2,4-difluorophenyl)-3-methylthio-1-(1H-1,2,4-triazol-1-yl)butan-2-ol, D-camphor-10-sulfonic acid and xylene being 1 : 1 : 0.4.

The above obtained (-)-threo-2-(2,4-difluorophenyl)-3-methylthio-1-(1H-1,2,4-triazol-1-yl)butan-2-ol D-camphor-10-sulfonate (1.50 g) was gradually added to a saturated aqueous sodium bicarbonate solution (4.0 ml) under stirring, and stirring was continued at 40° C for 1 hour. The reaction mixture was cooled to room temperature and the precipitated crystals were collected by filtration. The crystals were washed with water (5 ml) and dried under reduced pressure to give 0.74 g of (-)-threo-2-(2,4-difluorophenyl)-3-methylthio-1-(1H-1,2,4-triazol-1-yl)butan-2-ol, which was recrystallized from a mixture of toluene and heptane to give pure (-)-threo-2-(2,4-difluorophenyl)-3-methylthio-1-(1H-1,2,4-triazol-1-yl)butan-2-ol. Overall yield, 18.6 %.
Optical purity: 94 % e.e. (on HPLC analysis).
Melting point: 130 - 133° C.
$[\alpha]_D^{25}$ : -120.4° (c = 0.50, $CHCl_3$).

| Elementary analysis for $C_{13}H_{15}OF_2N_3S$ (%): | |
|---|---|
| Calcd.: | C, 52.16; H, 5.05; N, 14.04; S, 10.71. |
| Found: | C, 52.13; H, 5.06; N, 14.09; S, 11.1. |

Reference Example 5

Optical resolution of (±)-threo-2-(2,4-difluorophenyl)-3-methylthio-1-(1H-1,2,4-triazol-1-yl)butan-2-ol:

(±)-threo-2-(2,4-Difluoro)-3-methylthio-1-(1H-1,2,4-triazol-1-yl)butan-2-ol (898 mg), 3-bromo-d-camphor-8-sulfonic acid monohydrate (988 mg) and toluene (10 ml) were charged in a reactor, and the resultant mixture was heated for dissolution of crystals under reflux. The reaction mixture was cooled to 40° C. Seed crystals of high purity were inoculated thereto at the same temperature, and the resultant mixture was stirred at room temperature for 3 hours for precipitation of crystals. The precipitated crystals were collected by filtration, washed with toluene (7 ml) and dried under reduced pressure to give 713 mg of (-)-threo-2-(2,4-difluorophenyl)-3-methylthio-1-(1H-1,2,4-triazol-1-yl)butan-2-ol 3-bromo-d-camphor-8-sulfonate.
Optical purity: 89 % e.e. (on HPLC analysis).
Melting point: 162 - 165° C (decomp.).
$[\alpha]_D^{25}$ : -10.0° (c = 0.50, $CHCl_3$).

The filtrate was concentrated to give (+)-threo-2-(2,4-difluorophenyl)-3-methylthio-1-(1H-1,2,4-triazol-1-yl)butan-2-ol 3-bromo-d-camphor-8-sulfonate.
Optical purity: 56 % e.e. (on HPLC analysis).
$[\alpha]_D^{25}$ : +78.3° (c = 0.50, $CHCl_3$).

The thus obtained crystals (684 mg)of the (-)-isomer was combined with toluene (5.5 ml), heated at 50° C for 2 hours and cooled to room temperature, followed by collection of crystals by filtration. The

precipitated crystals were washed with toluene (5 ml) and dried under reduced pressure to give 622 mg of (-)-threo-2-(2,4-difluorophenyl)-3-methylthio-1-(1H-1,2,4-triazol-1-yl)butan-2-ol 3-bromo-d-camphor-8-sulfonate. The nuclear magnetic resonance spectrum revealed that the molar ratio of the base and the acid in this product was 1 : 1.

Optical purity: 99 % e.e. (on HPLC analysis).
Melting point: 167 - 169 ° C (decomp.).
$[\alpha]_D^{25}$ : -17.9 ° (c = 0.50, $CHCl_3$).

The above obtained (-)-threo-2-(2,4-difluorophenyl)-3-methylthio-1-(1H-1,2,4-triazol-1-yl)butan-2-ol 3-bromo-d-camphor-8-sulfonate (400 mg) was gradually added to a saturated aqueous sodium bicarbonate solution (1.6 ml) under stirring, and stirring was continued at 40 ° C for 1 hour. The reaction mixture was cooled to room temperature and the precipitated crystals were collected by filtration. The crystals were washed with water (2 ml) and dried under reduced pressure to give 190 mg of (-)-threo-2-(2,4-difluorophenyl)-3-methylthio-1-(1H-1,2,4-triazol-1-yl)butan-2-ol, which was recrystallized from a mixture of toluene and heptane to give pure (-)-threo-2-(2,4-difluorophenyl)-3-methylthio-1-(1H-1,2,4-triazol-1-yl)butan-2-ol. Overall yield, 29.6 %.

Optical purity: >99 % e.e. (on HPLC analysis).
Melting point: 134.5 - 135.0 ° C.
$[\alpha]_D^{25}$ : -126.1 ° (c = 0.50, $CHCl_3$).

| Elementary analysis for $C_{13}H_{15}OF_2N_3S$ (%): | |
|---|---|
| Calcd.: | C, 52.16; H, 5.05; N, 14.04; S, 10.71. |
| Found: | C, 52.00; H, 5.08; N, 14.01; S, 10.4. |

## Claims

1. A method for the optical resolution of sulfone derivatives, which comprises reacting a mixture of the d- and 1-isomers of a sulfone derivative of the formula:

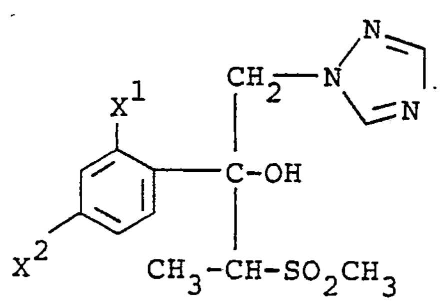

(I)

wherein $X^1$ and $X^2$ are each and independently a hydrogen atom or a halogen atom with optically active 10-camphorsulfonic acid to make a mixture of two kinds of diastereomer salts between them, isolating one of the diastereomer salts from said diastereomer salt mixture on the difference of the diastereomer salts in solubility and decomposing the isolated diastereomer salt.

2. The method according to claim 1, wherein the other diastereomer salt is recovered from the mother liquor after the isolation of the diastereomer salt, the recovered salt is decomposed to the corresponding sulfone derivative (I) and the sulfone derivative (I) is reacted with optically active 10-camphorsulfonic acid having an optical rotation opposite to that of the optically active 10-camphorsulfonic acid as previously used.

3. The method according to claim 1, wherein the sulfone derivative (I) is (±)-threo-2-(2,4-difluorophenyl)-3-methylsulfonyl-1-(1H-,1,2,4-triazol-1-yl)butan-2-ol.

4. A salt between an optically active isomer of the sulfone derivative (I) and optically active 10-camphorsulfonic acid.

5. The salt according to claim 4, wherein the sulfone derivative (I) is (-)-threo-2-(2,4-difluorophenyl)-3-methylsulfonyl-1-(1H-,1,2,4-triazol-1-yl)butan-2-ol and the optically active 10-camphorsulfonic acid is D-10-

camphorsulfonic acid.

Claims for the folowing Contracting State: ES

1. A method for the optical resolution of sulfone derivatives, which comprises reacting a mixture of the d- and 1-isomers of a sulfone derivative of the formula:

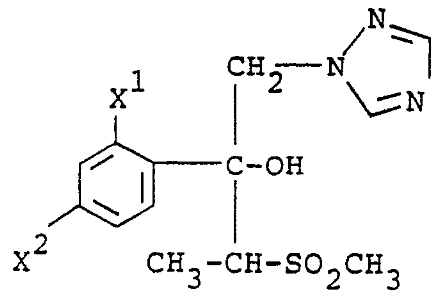

(I)

wherein $X^1$ and $X^2$ are each and independently a hydrogen atom or a halogen atom with optically active 10-camphorsulfonic acid to make a mixture of two kinds of diastereomer salts between them, isolating one of the diastereomer salts from said diastereomer salt mixture on the difference of the diastereomer salts in solubility and decomposing the isolated diastereomer salt.

2. The method according to claim 1, wherein the other diastereomer salt is recovered from the mother liquor after the isolation of the diastereomer salt, the recovered salt is decomposed to the corresponding sulfone derivative (I) and the sulfone derivative (I) is reacted with optically active 10-camphorsulfonic acid having an optical rotation opposite to that of the optically active 10-camphorsulfonic acid as previously used.

3. The method according to claim 1, wherein the sulfone derivative (I) is (±)-threo-2-(2,4-difluorophenyl)-3-methylsulfonyl-1-(1H-,1,2,4-triazol-1-yl)butan-2-ol.